(19) Europäisches Patentamt

European Patent Office

Office européen des brevets

(11) Veröffentlichungsnummer: **0 288 945 B1**

## (12) EUROPÄISCHE PATENTSCHRIFT

(45) Veröffentlichungstag der Patentschrift: **30.12.92**

(21) Anmeldenummer: **88106560.1**

(22) Anmeldetag: **23.04.88**

Die Akte enthält technische Angaben, die nach dem Eingang der Anmeldung eingereicht wurden und die nicht in dieser Patentschrift enthalten sind.

(51) Int. Cl.⁵: **A61K 47/36**, A61K 6/00

(54) **Grundlage für Schleimhaut- und Prothesenhaft-Pasten, Verfahren zu ihrer Herstellung sowie Pasten auf Basis dieser Grundlage.**

(30) Priorität: **28.04.87 DE 3714074**

(43) Veröffentlichungstag der Anmeldung:
**02.11.88 Patentblatt 88/44**

(45) Bekanntmachung des Hinweises auf die Patenterteilung:
**30.12.92 Patentblatt 92/53**

(84) Benannte Vertragsstaaten:
**AT CH DE LI**

(56) Entgegenhaltungen:
**EP-A- 0 139 913**
**FR-A- 1 367 858**
**US-A- 3 930 871**

(73) Patentinhaber: **HOECHST AKTIENGESELL-SCHAFT**
**Postfach 80 03 20**
**W-6230 Frankfurt am Main 80(DE)**

(72) Erfinder: **Reul, Bernhard**
**An den Geierwiesen 20**
**W-6240 Königstein/Taunus(DE)**
Erfinder: **Petri, Walter, Dr.**
**Panoramastrasse 19**
**W-6272 Niedernhausen/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Grundlage für Schleimhautpasten und Prothesenhaftpasten sowie ein Verfahren zu ihrer Herstellung.

In der US Patentschrift 3,930,871 werden Prothesenhaftmittel auf Basis von Dextrin, das wasserlöslich ist, und Xanthomonas campestris hydrophilic colloid ((R)Keltrol F) beschrieben.

Gelier- und Verdickungsmittel auf Basis von Cassia-Galactomannanen, die eine synergistische Mischung aus u.a. Carrageenan, Agar und/oder Xanthan enthalten, sind aus EP-A-0 139 913 bekannt, wobei für Mischungen aus Guar/Xanthan angegeben ist, daß diese keinerlei Gelbildung zeigen.

Eine Zubereitung (Paste), bei der eine Schleimhautsalbengrundlage entsprechend der Deutschen Patentschrift 1 275 729 (= GB Patent 1050967) verwendet wird, zeigt bereits nach kurzer Lagerzeit Separierung der flüssigen Phase (= Ausbluten), wenn sie Wirk- und Hilfsstoffe mit oberflächenaktiven Eigenschaften enthält.

Die Flüssigkeitsseparierung beruht darauf, daß die Viskosität der inneren Phase der Paste nicht hinreichend erhöht werden kann, um das Ausbluten zu verhindern. Selbst durch eine mengenmäßige Steigerung des nur begrenzt kolloidal löslichen Anteils an niedermolekularem Celluloseether oder an Natriumalginat kann ein Ausbluten daher nicht vermieden werden.

Weiterhin können Calcium-Salze, z.B. in Form von Glycerin-Addukten, die zur Konsistenzverbesserung von Schleimhaut- und Prothesenhaft-Pasten, zur Steigerung des Haftvermögens, als die Wirkstofffreigabe beeinflussende Substanzen bzw. als Calciumspender in modernen Mundheilpasten eingesetzt werden, in die beschriebene Grundlage, die Natriumalginat enthält, nicht eingearbeitet werden, da nach dem Auftragen auf die Schleimhaut in Wasser schwerlösliche Ausfällungen von Calciumalginaten auftreten.

Diese Ausfällungen rufen eine starke Verfestigung der Paste hervor, verbunden mit einer erheblichen Verminderung von Haftwirkung und Haftdauer. Pasten dieser Art entsprechen nicht den üblichen Anforderungen an Schleimhaut- und Prothesenhaftpasten.

Es wurde nun gefunden, daß die beschriebenen Nachteile vermieden werden können, wenn man in der Grundlage gemäß Deutscher Patentschrift 1 275 729 die kolloidale Lösung von niedermolekularen Celluloseethern oder Natriumalginat ersetzt durch eine kolloidale Lösung des linearen, physiologisch gut verträglichen Xanthan Gum. Dieser Stoff löst sich kolloidal in der inneren Pastenphase, z.B. in Glycerin, auf und führt bereits in geringen Konzentrationen zu signifikanten Viskositätserhöhungen.

Die Erfindung betrifft daher eine Grundlage für Schleimhaut- und Prothesenhaft-Pasten, enthaltend einen nur in Wasser quellbaren, festen Quellstoff, dispergiert in einer kolloidalen Lösung eines in Wasser quellbaren, organischen zweiten Quellstoffs in einem wasserlöslichen, physiologisch verträglichen, organischen Lösungsmittel, die dadurch gekennzeichnet ist, daß der organische zweite Quellstoff der ionogene Quellstoff Xanthan Gum ist und das organische Lösungsmittel weniger als 1 % Wasser enthält.

Die Erfindung betrifft ferner ein Verfahren zur Herstellung einer Grundlage für Schleimhaut- und Prothesenhaft-Pasten, welches dadurch gekennzeichnet ist, daß man der kolloidalen Lösung von Xanthan Gum in einem wasserlöslichen, organischen, physiologisch verträglichen Lösungsmittel, das weniger als 1 % Wasser enthält, einen in der genannten kolloidalen Lösung nicht quellenden, in Wasser jedoch quellbaren festen Quellstoff, zusetzt.

Es ist vorteilhaft, wenn die zu dispergierenden bzw. zu lösenden Quellstoffe in feinverteilter Form vorliegen. Feinverteiltes Xanthan Gum erhält man z.B. durch vorherige Mahlung in Ethanol. An Stelle der Mahlung kann das Xanthan Gum in dem wasserlöslichen organischen, physiologisch verträglichen Lösungsmittel unter Erhitzen, z.B. auf 50° bis 80°C gelöst werden; nach Abkühlung der Lösung werden die weiteren Substanzen zugesetzt.

Eine für die Aufnahme des wasserquellbaren festen Quellstoffs als Grundlage geeignete kolloidale Lösung erhält man aus dem Xanthan Gum und einem wasserlöslichen, physiologisch verträglichen organischen Lösungsmittel. Als solche sind z.B. verwendbar flüssige, mehrwertige Alkohole, insbesondere Glycerin. Die Lösungsmittel enthalten weniger als 1 % Wasser, d.h. sie sind im wesentlichen wasserfrei, damit der in der Paste ungequollene aber wasserquellbare Quellstoff nicht schon in der Paste anquillt.

Als wasserquellbare in der kolloidalen Lösung dispergierbare Quellstoffe eignen sich Polysaccharide wie z.B. Tragant, Johannisbrotkernmehl sowie Galactomannane, insbesondere Guaranpräparate,wie Guar Gum (= Guaran). Es lassen sich besonders mit Guaranprodukten weiche, schmiegsame Pasten herstellen, die die Feuchtigkeit schnell aufnehmen, rasch die Struktur eines festen Filmes annehmen und die in der Pastengrundlage gegebenenfalls inkorporierten Arzneistoffe verzögert an die Schleimhaut der Applikationsstelle abgeben.

Die erfindungsgemäße Grundlage ist geschmeidig, gut auftragbar und zeichnet sich durch eine gute und lang andauernde Haftfähigkeit aus. Sie eignet sich als Grundlage für Prothesenhaftpasten, Schleimhaut- und Schleimhautheilpasten wie Wundabdeckpasten, Hämorrhoidal-und Vaginalpasten, insbesondere als Grundlage für Pasten zur Behandlung der Mundhöhlenschleimhaut. Die Erfindung betrifft daher auch Schleimhaut- und Prothesenhaft-Pasten auf Basis der erfindungsgemäßen Grundlage.

Bringt man eine mit einer erfindungsgemäßen Grundlage aufgebaute Paste z.B. auf die Mundschleimhaut, so haftet sie fest daran. Der in der Paste enthaltene ungequollene Quellstoff wie z.B. Guaran nimmt über das wasserlösliche, physiologisch verträgliche organische Lösungsmittel sofort die Feuchtigkeit der Schleimhautoberfläche auf. Der Pastenfilm wird dadurch auf der Schleimhautoberfläche fixiert und verfestigt sich durch den nachquellenden Quellstoff.

Es war überraschend, daß die durch das ionogene Polysaccharid Xanthan Gum hervorgerufene Viskositätserhöhung auch nach Applikation auf der Schleimhaut nach Wasserzutritt im Gegensatz zu einer durch Zelluloseether hervorgerufenen Viskositätserhöhung erhalten bleibt und je nach der eingesetzten Konzentration (Menge) gegebenenfalls sich noch verstärkt. Nicht vorhersehbar war, daß das in der inneren Pastenphase kolloidal gelöste Polysaccharid zu einer deutlich verbesserten Soforthaftwirkung und Haftdauer der Paste an der Schleimhaut führt. Dieses Erscheinungsbild des ionogenen Polysaccharids Xanthan Gum war im Vergleich zum Kenntnisstand der Quellbarkeit/Löslichkeit von Polysacchariden in Glycerin bzw. Wasser und auch im Vergleich zum Kenntnisstand der Eigenschaften von Zelluloseethern überraschend.

In die Grundlage können prizipiell alle lokal anwendbaren Wirkstoffe eingearbeitet werden. Wirkstoffe folgender Gruppen kommen beispielsweise in Betracht: Antiphlogistika, Antibiotika, Antiseptika, Lokalanesthetika, Antihistaminika, Antimykotika, Steroid-Hormone, Antiparadontosemittel, Chemotherapeutika, Enzyme sowie Calciumspender.

Als Hilfsstoffe können beispielsweise eingearbeitet werden: übliche Hilfsstoffe, wie z.B. kolloidale Kieselsäure, Hydroxyethylcellulose oder auch Calciumacetat- oder Calciumlactat-Glycerin-Addukt (in der deutschen Patentanmeldung P 37 10 177.3 (HOE 87/F 090) wird Calciumlactat-Glycerin-Addukt für die Verwendung als Hilfsstoff vorgeschlagen); ferner können pharmakologisch unbedenkliche Geruchs- und Geschmackskorrigentien wie z.B. Saccharin-Na oder Farbstoffe wie z.B. Carmin, eingearbeitet werden.

Die folgenden Beispiele erläutern die Erfindung. Hierzu werden in die kolloidale Lösung von Xanthan Gum die übrigen Bestandteile unter Rühren und gegebenenfalls Vakuum eingearbeitet.

**Beispiel 1**

### 1 g Wundabdeckpaste enthält:

| | |
|---|---:|
| Xanthan Gum | 5,0 mg |
| Guar Gum | 225,0 mg |
| Glycerin, wasserfrei | 770,0 mg |
| | 1000,0 mg |

**Beispiel 2**

### 1 g Prothesenhaftpaste enthält:

| | |
|---|---:|
| Didecyldimethylammonium-chlorid | 1,0 mg |
| Xanthan Gum | 3,0 mg |
| Guar Gum | 250,0 mg |
| Carmin | 0,5 mg |
| Glycerin, wasserfrei | 745,5 mg |
| | 1000,0 mg |

**Beispiel 3**

1 g Mundheilpaste enthält:

| | |
|---|---|
| Prednisolon-21-acetat | 5,58 mg |
| (entspr. 5 mg Prednisolon) | |
| Neomycin-HCl | 4,37 mg |
| (entspr. 3,5 mg Base) | |
| Aminoquinurid-HCl, 3,5 $H_2O$ | 3,00 mg |
| Calciumacetat-Glycerin-Addukt | 175,00 mg |
| Xanthan Gum | 1,00 mg |
| Guar Gum | 225,00 mg |
| Glycerin, wasserfrei | 586,05 mg |
| | 1000,00 mg |

**Beispiel 4**

1 g Mundheilpaste enthält:

| | |
|---|---|
| Didecyldimethylammonium-chlorid | 2,00 mg |
| Lidocain-HCl·$H_2O$ | 5,33 mg |
| Calciumlactat-Glycerin-Addukt | 200,00 mg |
| Kieselsäure, kolloidal | 1,75 mg |
| Xanthan Gum | 1,00 mg |
| Guar Gum | 250,00 mg |
| Saccharin-Na | 1,00 mg |
| Carmin | 0,50 mg |
| Glycerin, wasserfrei | 538,42 mg |
| | 1000,00 mg |

**Beispiel 5**

4

1 g Mundheilpaste enthält:

| Calcium-glycerinphosphat·$H_2O$ | 125,83 mg |
| Xanthan Gum | 2,00 mg |
| Guar Gum | 200,00 mg |
| Saccharin-Na | 0,50 mg |
| Carmin | 0,50 mg |
| Glycerin, wasserfrei | 671,17 mg |
| | 1000,00 mg |

Beispiel 6

1 g Hämorrhoidalpaste enthält:

| Fluorometholon | 0,20 mg |
| Neomycin-HCl | 2,00 mg |
| Aminoquinurid-HCl·3,5 $H_2O$ | 3,00 mg |
| Pheniramin-HCl | 11,52 mg |
| Xanthan Gum | 2,00 mg |
| Guar Gum | 250,00 mg |
| Glycerin, wasserfrei | 731,28 mg |
| | 1000,00 mg |

Beispiel 7

1 g Hämorrhoidalpaste enthält:

| Chlorhexidin-2HCl | 2,00 mg |
| Lidocain-HCl·$H_2O$ | 5,33 mg |
| Allantoin | 5,00 mg |
| Dexpanthenol | 5,00 mg |
| Azulen | 2,00 mg |
| Xanthan Gum | 3,00 mg |
| Guar Gum | 225,00 mg |
| Glycerin, wasserfrei | 752,67 mg |
| | 1000,00 mg |

**Beispiel 8**

### 1 g Vaginalpaste enthält:

| | |
|---|---|
| Ciclopirox | 7,71 mg |
| Xanthan Gum | 3,00 mg |
| Guar Gum | 100,00 mg |
| Glycerin, wasserfrei | 889,29 mg |
| | 1000,00 mg |

**Beispiel 9**

### 1 g Vaginalpaste enthält:

| | |
|---|---|
| Estriol | 0,01 mg |
| Benzalkoniumchlorid | 2,00 mg |
| Xanthan Gum | 2,50 mg |
| Guar Gum | 200,00 mg |
| Glycerin, wasserfrei | 795,49 mg |
| | 1000,00 mg |

**Beispiel 10**

### 1 g Parodontoseheilpaste enthält:

| | |
|---|---|
| Dicalciumhydrogenphosphat·$2H_2O$ | 100,00 mg |
| Natriummonohydrogenphosphat·$2H_2O$ | 5,00 mg |
| Magnesium-ammoniumphosphat·$6H_2O$ | 10,00 mg |
| Natriumfluorid (entspr. 2 mg Fluor) | 3,65 mg |
| Xanthan Gum | 1,00 mg |
| Guar Gum | 200,00 mg |
| Glycerin, wasserfrei | 680,35 mg |
| | 1000,00 mg |

**Patentansprüche**

1. Grundlage für Schleimhaut- und Prothesenhaft-Pasten, enthaltend einen nur in Wasser quellbaren, festen Quellstoff, dispergiert in einer kolloidalen Lösung eines in Wasser quellbaren, organischen

6

zweiten Quellstoffs in einem wasserlöslichen, physiologisch verträglichen, organischen Lösungsmittel, dadurch gekennzeichnet, daß der organische zweite Quellstoff der ionogene Quellstoff Xanthan Gum ist und das organische Lösungsmittel weniger als 1% Wasser enthält.

2. Schleimhautpaste enthaltend eine Grundlage gemäß Anspruch 1 und lokal anwendbare Wirkstoffe und gegebenenfalls übliche Hilfsstoffe.

3. Schleimhautpaste enthaltend eine Grundlage gemäß Anspruch 1, übliche Hilfsstoffe und einen, zwei oder drei Wirkstoffe ausgewählt aus der Gruppe bestehend aus Antiphlogistika, Antibiotika, Antiseptika, Localanesthetika, Antihistaminika, Antimykotika, Steroid-Hormone, Antiparodontosemittel, Chemotherapeutika, Enzyme und Calciumspender.

4. Scheimhautpaste zur Anwendung an der Mundhöhlenschleimhaut enthaltend eine Grundlage gemäß Anspruch 1, übliche Hilfsstoffe und ein, zwei oder drei Wirkstoffe ausgewählt aus der Gruppe bestehend aus Antiphlogistika, Antibiotika, Antiseptika, Lokalanesthetika, Antiparodontosemittel und Calciumspender.

5. Prothesenhaftpaste enthaltend eine Grundlage gemäß Anspruch 1 und lokal anwendbare Wirkstoffe und gegebenenfalls übliche Hilfsstoffe.

6. Verfahren zur Herstellung einer Grundlage gemäß Anspruch 1, dadurch gekennzeichnet, daß man der kolloidalen Lösung von Xanthan Gum in einem wasserslöslichen, organischen, physiologisch verträglichen Lösungsmittel, das weniger als 1% Wasser enthält, einen in der genannten kolloidalen Lösung nichtquellenden, in Wasser jedoch quellbaren festen Quellstoff zusetzt.

**Claims**

1. A base for mucosal and denture adhesive pastes, containing a solid swelling agent which is swellable only in water and is dispersed in a colloidal solution of an organic second swelling agent, which is swellable in water; in a water-soluble, physiologically tolerated organic solvent, wherein the organic second swelling agent is the ionic swelling agent xanthan gum and the organic solvent contains less than 1% water.

2. A mucosal paste containing a base as claimed in claim 1 and active substances which can be used topically and, where appropriate, customary auxiliaries.

3. A mucosal paste containing a base as claimed in claim 1, customary auxiliaries and one, two or three active substances selected from the group comprising antiinflammatory agents, antibiotics, antiseptics, local anesthetics, antihistamines, antimycotics, steroid hormones, antiperiodontosis agents, chemotherapeutics, enzymes and calcium suppliers.

4. A mucosal paste for use on the oral mucosa, containing a base as claimed in claim 1, customary auxiliaries and one, two or three active substances selected from the group comprising antiinflammatory agents, antibiotics, antiseptics, local anesthetics, antiperiodontosis agents and calcium suppliers.

5. A denture adhesive paste containing a base as claimed in claim 1 and active substances which can be used topically and, where appropriate, customary auxiliaries.

6. A process for the preparation of a base as claimed in claim 1, which comprises addition of a solid swelling agent which does not swell in the said colloidal solution but is swellable in water to the colloidal solution of xanthan gum in a water-soluble organic physiologically tolerated solvent which contains less than 1% water.

**Revendications**

1. Matière de base pour pâtes pour muqueuses et pâtes adhérentes aux prothèses, contenant un agent de gonflement solide ne gonflant que dans l'eau, dispersé dans une solution colloïdale d'un deuxième

7

agent de gonflement organique gonflant dans l'eau dans un solvant organique hydrosoluble physiologiquement compatible, caractérisé en ce que le deuxième agent de gonflement organique consiste en l'agent de gonflement ionogène constitué par la gomme xanthane et le solvant organique contient moins de 1% d'eau.

2. Pâte pour muqueuses contenant une matière de base selon la revendication 1 et un principe actif à usage-local, et éventuellement des additifs usuels.

3. Pâte pour muqueuses contenant une matière de base selon la revendication 1, des additifs usuels et un, deux ou trois principes actifs choisis parmi les anti-inflammatoires, les antibiotiques, les antiseptiques, les anesthésiques locaux, les anti-histaminiques, les antimycosiques, les hormones stéroïdiennes, les agents anti-parodontose, les agents chimio-thérapeutiques, les enzymes et les agents dispensant du calcium.

4. Pâte pour muqueuses pour application sur les muqueuses de la cavité buccale contenant une matière de base selon la revendication 1, des additifs usuels et un, deux ou trois principes actifs choisis parmi les anti-inflammatoires, les antibiotiques, les antiseptiques, les anesthésiques locaux, les agents anti-parodontose et les agents dispensant du calcium.

5. Pâte adhérant aux prothèses contenant une matière de base selon la revendication 1 et un principe actif à usage local, et éventuellement des additifs usuels.

6. Procédé de préparation d'une matière de base selon la revendication 1, caractérisé en ce que l'on ajoute à la solution colloïdale de gomme xanthane dans un solvant organique hydrosoluble physiologiquement compatible, contenant moins de 1% d'eau, un agent de gonflement solide ne gonflant pas dans ladite solution colloïdale, mais gonflant cependant dans l'eau.